# EUROPEAN PATENT SPECIFICATION

(11) **EP 0 393 196 B1**
(45) Date of publication and mention of the grant of the patent: **02.11.1995**
(21) Application number: 89907277.1
(22) Date of filing: 15.06.1989
(51) Int. Cl.: G01N 21/27, G01N 30/74, G01N 30/88

(54) **MOLECULAR WEIGHT DISTRIBUTION MEASUREMENT METHOD, SPECTROPHOTODETECTOR AND MOLECULAR WEIGHT DISTRIBUTION MEASUREMENT APPARATUS OF POLYMERS**
MESSVERFAHREN DER VERTEILUNG DER MOLEKÜLGEWICHTE, SPEKTRUMSPHOTODETEKTOR UND MESSVERFAHREN DER VERTEILUNG DER MOLEKÜLGEWICHTE BEI POLYMEREN
PROCEDE DE MESURE DE LA DISTRIBUTION DU POIDS MOLECULAIRE, SPECTROPHOTODETECTEUR ET APPAREIL DE MESURE DE LA DISTRIBUTION DU POIDS MOLECULAIRE DE POLYMERES

(30) Priority: 15.06.1988 JP 78345/88 U; 15.07.1988 JP 175100/88
(43) Date of publication of application: 24.10.1990
(73) Proprietor: IDEMITSU PETROCHEMICAL COMPANY LIMITED, Tokyo 100 (JP); SOMA OPTICS, Ltd., Tokyo (JP)
(72) Inventor: HOUSAKI, Tatsuya, c/o Idemitsu Petrochem. Co., Ltd, Chiba 299-01 (JP)
(74) Representative: Hoeger, Stellrecht & Partner
(86) International application number: JP8900601
(87) International publication number: WO8912814

(56) References cited:
- EP-A- 0 089 157
- JP-B- 0 627 975
- US-A- 3 287 557
- US-A- 3 692 415
- US-A- 4 192 614
- US-A- 4 532 043

## Description

The present invention relates to a method for measuring a molecular weight distribution according to the preamble of claim 1, to a spectrophotodetector according to the preamble of claim 2 and an apparatus for measuring a molecular weight distribution of polymers according to the preamble of claim 3.

The method, the spectrophotodetector and the apparatus according to the preambles of claims 1 to 3, respectively, are known from US-A-4 192 614 and US-A-4 532 043. More specifically, US-A-4 192 614 discloses a spectrophotodetector comprising a cell into which a liquid sample is introduced; a spectrophotodetecting member arranged apart from the cell; means to collect radiation from the cell on the photodetector that is light transmitting means; and means to provide a heat sink effect that is means for adjusting a temperature of the sample in the cell. Further, US-A-4 532 043 discloses an apparatus for measuring a distribution of a molecular weight of polymers comprising an eluent reservoir that is means for introducing polymers as a sample; a separating column containing a porous bed of stationary phase; a detector cell.

Generally, a chemical analysis by liquid chromatography is widely well-known. Recently, an apparatus having an improved pressure resistance, i.e., a high performance liquid chromatography, which can deliver an elute at a high flow velocity, has been developed to reduce a time necessary for analysis. The apparatus is widely used for the analysis of various samples.

A conventional spectrophotodetector used as a detecting member in the high performance liquid chromatography is composed of a cell forming a chamber for measuring a sample, a light source, and a light detector. The latter two members are arranged near and on each side of the cell, respectively, and a light from the light source directly irradiates the cell, permeates the sample in the cell, and is received by the light detector as an information signal for an analysis treatment.

In liquid chromatography, the sample is supplied to the cell in the form of a solution in an ionizing solvent. However, there are many samples which are not melted until the temperature is raised to as high as 150°C.

When a sample having a high temperature is introduced into the cell of a conventional spectrophotodetector as mentioned above, electric and optical systems, such as the light source and the light detector arranged near the cell cannot withstand the heat from the cell, and therefore, such a sample having a high temperature cannot be measured by a conventional spectrophotodetector.

An apparatus using a differential refractometer as a detector in the high performance liquid chromatography is also known. However, such an apparatus, which is currently commercially available, has a heat resistance of at most 150°C, even if a special solvent, such as sulfuric acid, is not used. Thus, a substance having a melting point of above 150°C cannot be measured by that apparatus.

To improve the above apparatus, Stacy et. al., proposed a method wherein polyphenylene sulfide (hereinafter referred to as PPS) is size-separated at a column temperature of 210°C, using alpha-chloronaphthalene, and a pressure difference between an inlet and an outlet of a capillary directly connected thereto is continuously measured (Journal Applied Polymer Science, 32, 3959, 1986). A viscosity of a polymer solution, however, is proportional to a molecular weight of the polymer. Thus, a higher molecular weight portion generates a strong signal, but a lower molecular weight portion generates a weak one. Therefore, it is difficult to perform an accurate measurement of the molecular weight distribution by the above method.

JP-U-63-135857 (A) discloses a GPC for a high temperature measurement having an FID. In this process, an amount of polymers remaining after vaporizing a solvent is measured. Because a complete vaporization of the solvent is difficult, an accurate determination of the molecular weight distribution is difficult. Further, a solvent harmful to humans is vaporized, and thus, an environmental pollution occurs.

To remedy the above problems, the present invention has been completed. An object of the present invention is to provide a method for measuring a molecular weight distribution wherein a substance which is not melted until the temperature is raised to as high as, for example, 150°C, can be easily and accurately analyzed, and environmental pollution does not occur, a spectrophotodetector for carrying out the above method, and an apparatus for measuring a distribution of a molecular weight of polymers.

This object according to the present invention is accomplished by means of the above referenced method, spectrophotometer and apparatus comprising the characterizing features of claims 1 to 3, respectively.

Thus, the present invention relates to a method for measuring a molecular weight distribution by receiving, at a light detecting member, a light permeated through a liquid sample in a cell, and analyzing the sample on the basis of a signal of the received light, characterized in that the sample passing through the cell is heated to an extent such that the sample is not sedimented, and the light which has been passed through the sample is received without the influence of a temperature of the cell and/or the sample.

Further, the present invention also relates to a spectrophotodetector comprising: a cell into which a liquid sample is introduced, a spectrophotodetecting member which is arranged apart from the cell to an extent such that the member is not affected by heat generated from the sample in the cell, a light transmitting means which optically connects the spectrophotodetecting member and the cell, and a means for adjusting a temperature of the sample in the cell.

Still further, the present invention also relates to an apparatus for measuring a distribution of a molecular weight of polymers, comprising: a means for supplying an elute, a means for introducing polymers as a sample into the supplied elute, a column which contains a packing material therein and which separates and delivers the elute containing the polymers, a cell into which the elute containing the polymers is introduced from the column, a spectrophotodetecting member which is arranged apart from the cell to an extent such that the member is not affected by heat generated from at least a polymer-containing elute introduced into the cell, a light transmitting means which optically connects the spectrophotodetecting member and the cell, and a means for adjusting a temperature of the sample in the cell.

Embodiments of the present invention will be explained hereinafter, with reference to the accompanied drawings, wherein
Figure 1 illustrates a structure of an embodiment of a spectrophotodetector according to the present invention;
Figure 2 illustrates a structure of an embodiment of an apparatus for measuring a distribution of a molecular weight of polymers, according to the present invention; and
Figures 3 and 4 are graphs of PPS elution curves.

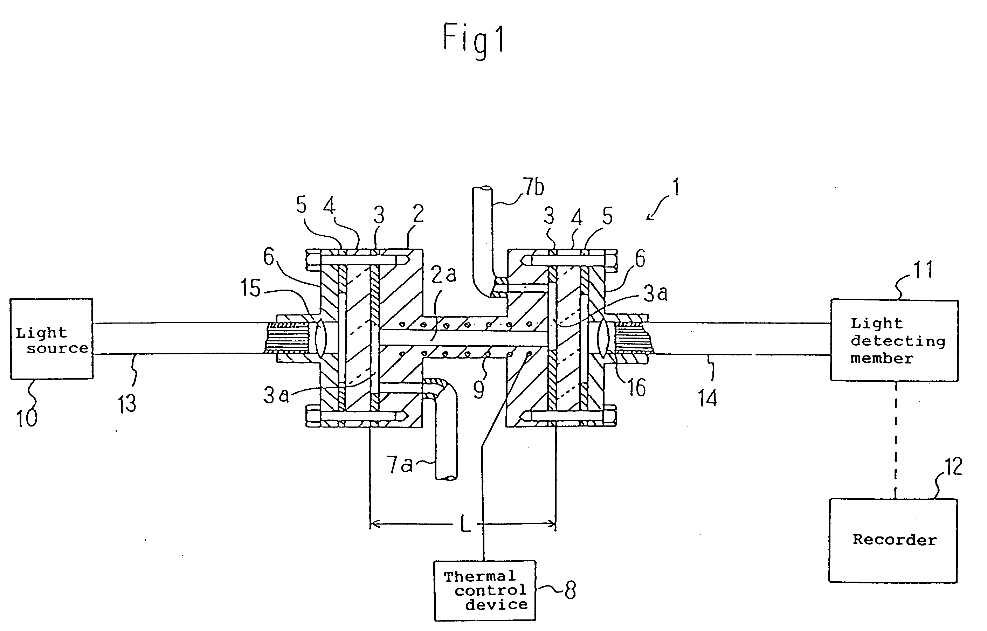

Referring to Fig. 1, an embodiment of a spectrophotodetector of the present invention will be described. Figure 1 shows a structure of one embodiment.

In Fig. 1, 1 denotes a cell as a measuring vessel. A liquid sample is continuously introduced into the cell, from a source (not shown) of supply of a sample, via a transfer tube. The cell 1 shown in Fig. 1 is an example of a Z type flow-through-cell, and comprises a body 2 including a hollow portion 2a, a gasket 3 having a slit 3a, a transparent window 4, an O-ring 5, and a fixing plate 6. A sample is introduced through a transfer tube 7a connected to the body 2, and driven into the hollow portion 2a in the body 2 via the slit 3a of one of the gaskets 3. Then, the sample is discharged through the slit 3a of the other gasket 3 and a transfer tube 7b.

The sample used in the present embodiment is a substance which is not melted until the temperature is raised to higher than 150°C. Therefore, the cell 1 must be made from a material capable of sufficiently withstanding such a high temperature. The body 2 and the fixing plate 6 of the cell 1 are made from a heat-resisting material, such as a stainless steel, and further, the transparent window 4 is made from a quartz or the like, and the gasket 3 and the O-ring 5 are made from a heat-resisting material such as a polyamide.

The cell 1 generally has an optical path (L) of 0.2 to 5.0 mm.

The cell 1 includes a heating device used to maintain the introduced sample at a constant temperature. An inside of the cell 1 can be maintained at a desired temperature (generally, 60 to 260°C) by operating a thermal control device 8. The thermal control device is, for example, a nichrome wire 9 embedded in the body 2, or an insulating vessel housing the entire cell 1.

As the cell 1, any cell other than the flow-cell may be used.

In Fig. 1, 10 is a light source. This light source is a hydrogen-discharge tube in the case of a measurement in an ultraviolet region, and a tungsten bulb in the case of a measurement in a visible light region, or the like. Further, 11 is a light detecting member and is composed of an apparatus such as a photoelectronmultiplier.

The light source 10 and the light detector 11 are contained in a single box, as a spectrophotodetecting member.

Also, 12 is a recorder which amplifies a signal from the light detector 11 and records the amplified signal for a time to output a chromatogram, and 13 and 14 are optical fibers, as an example of the light transmitting means, which optically connects the light source 10, light detector 11, and the transparent window 4 of the cell 1. The optical fibers 13 and 14 have a sufficient length such that a heat of the sample introduced into the cell 1 is not transmitted to the light source 10 and the light detector 11. Condensers 15 and 16 are arranged at connecting portions of the optical fibers 13 and 14, and the transparent windows 4 of the cell 1, condense the light from the light source 10 to the hollow portion 2a, and drive the light permeated therethrough to the optical fiber 14, without loss.

An example of a method for measuring a molecular weight distribution, according to the present invention, using the above-mentioned apparatus for measuring a molecular weight distribution will be explained hereinafter.

The sample (liquid) continuously introduced into the hollow portion 2a of the cell 1 from the transfer tube 7a is heated, to an extent such that the sample is not sedimented, by the thermal control device. The light from the light source 10 is irradiated through the optical fiber 13. The light which has not been absorbed by, but has been permeated through the sample, is received at the light detecting member 11 through the optical fiber 14. The recorder 12 outputs a chromatogram on the basis of a signal of the received light. The light source 10 and the light detecting member 11 are sufficiently separated from the cell 1 via the optical fibers 12 and 13, and thus a measurement can be carried out without an adverse effect of the heat generated from the sample and the cell 1.

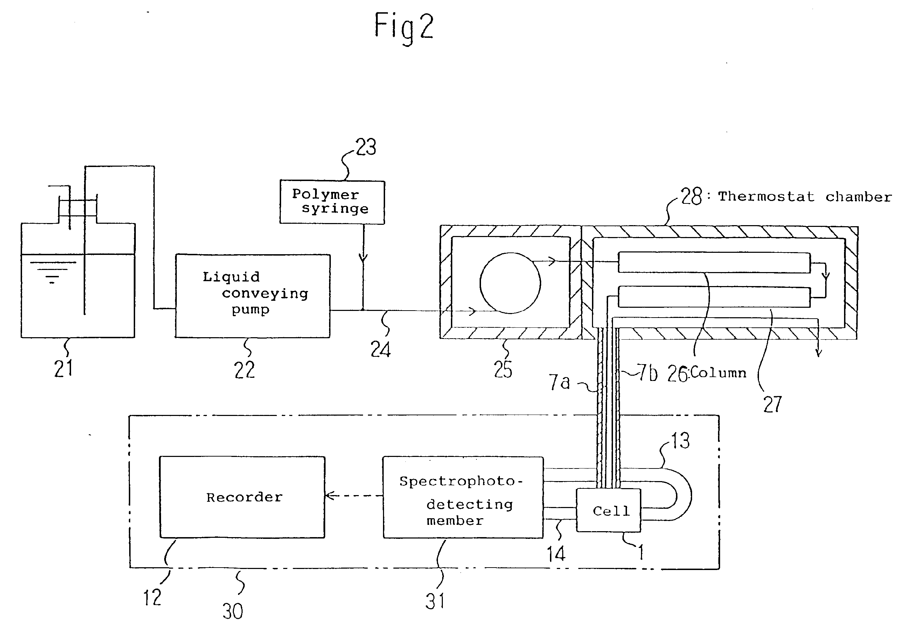

An apparatus of the present invention for measuring a distribution of a molecular weight of polymers will be explained hereinafter. This apparatus contains the above-mentioned spectrophotodetector. An example thereof will be described, with reference to Figure 2, which shows a structure thereof. In Fig. 2, portions the same as those shown in Fig. 1 are marked with the same symbols, and detailed explanations thereof are omitted.

In Fig. 2, 21 is a tank for supplying an elute, 22 is a pump for conveying a liquid, and 23 is a syringe holding polymers. A predetermined elute suitable for polymers as a sample is stored at a predetermined temperature in the tank 21, from which the elute is supplied. The elute is continuously supplied through a pipe 24 by the liquid-conveying pump 22, and a predetermined amount of polymers to be analyzed is introduced into the elute passing through the pipe 24 by the syringe 23 holding the polymers. The polymers are melted in the elute at a certain ratio.

In Fig. 2, 25 denotes a heater which raises a temperature of the elute (containing the polymers), which suffered a drop in temperature while being conveyed through the pipe 24, to a predetermined temperature, and 26 and 27 are columns which contain therein packing materials suitable for the samples, and separate the polymers in the elute which has passed through the heater 25. The columns 26 and 27 are maintained at a constant temperature in a thermostat chamber 28.

The polymers separated in the columns 26 and 27 in the elute are introduced into the cell 1 via a transfer tube 7a. If necessary, a thermal control device can be provided around the transfer tube 7a, to maintain a temperature therein.

In Fig. 2, 30 is a spectrophotodetector. The same spectrophotodetector as shown in Fig. 1 is used in the apparatus of Fig. 2. A spectrophotedetecting member 31 contains, in a single box, the light source 10 and the light detecting member 11 as shown in Fig. 1.

According to the apparatus of the present invention, for measuring a distribution of a molecular weight of polymers, the polymers melted in the elute are not sedimented while conveyed, because the polymers are maintained at a constant temperature by the heater 25 and the thermostat chamber 28. Further, because the spectrophotodetector 30 is sufficiently separated from the cell 1 via the optical fibers 13 and 14, and is not affected by the heat of the elute introduced into the cell 1, an accurate measurement can be carried out. Therefore, polymers which are not melted until an elevated temperature is reached, such as PPS, polyetheretherketone, polyethernitrile, and liquid crystal polyester, can be accurately measured.

The present invention is not limited by the above-mentioned embodiments.

For example, various flow-cells other than the Z type flow-cell shown in Fig. 1 may be employed, as long as the material thereof is heat-resistant.

The spectrophotodetector of the present invention, and the apparatus according to the present invention for measuring a distribution of a molecular weight of polymers, also may be used for an analysis of a sample which is melted at a temperature lower than 150°C.

A distribution of a molecular weight of the PPS was measured, using the apparatus as shown in Fig. 2.

### Example 1

As a sample, PPS (Lighten V-1; Phillips) was used. After the PPS was finely divided, 10 mg of the PPS was added to 5 ml of 1-chloronaphthalene to form an about 0.2% solution. After dissolved under heating, the solution was cooled to a room temperature, and the resulting homogeneous slurry was used. The slurry was introduced in an amount of 500 µl.

A pump SP8810 (manufactured by Spectra Physics), and an injector 7125 (manufactured by Leodyne) were used. The UV/VIS spectrophotodetector used was prepared by modifying an S3725 (Souma Kogaku). As a column, an AT80M/S (Showa Denko) was used. The UV/VIS spectrophotodetector, the column, and a portion connecting the column and the spectrophotodetector were heated at 210°C.

The solvent used was 1-chloronaphthalene, at a flow rate of 1 ml/min. The UV absorption wave length was 356 nm.

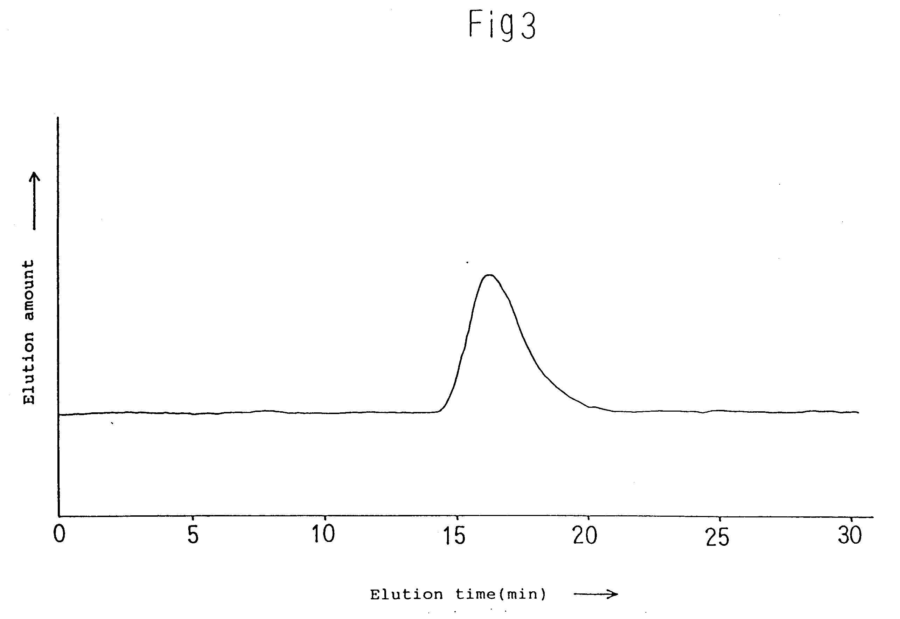

As a result, an elution curve as shown in Fig. 3 was obtained.

### Example 2

The procedure as mentioned in Example 1 was repeated, except that W-205 (Kureha Chemical Industry) was used as the PPS.

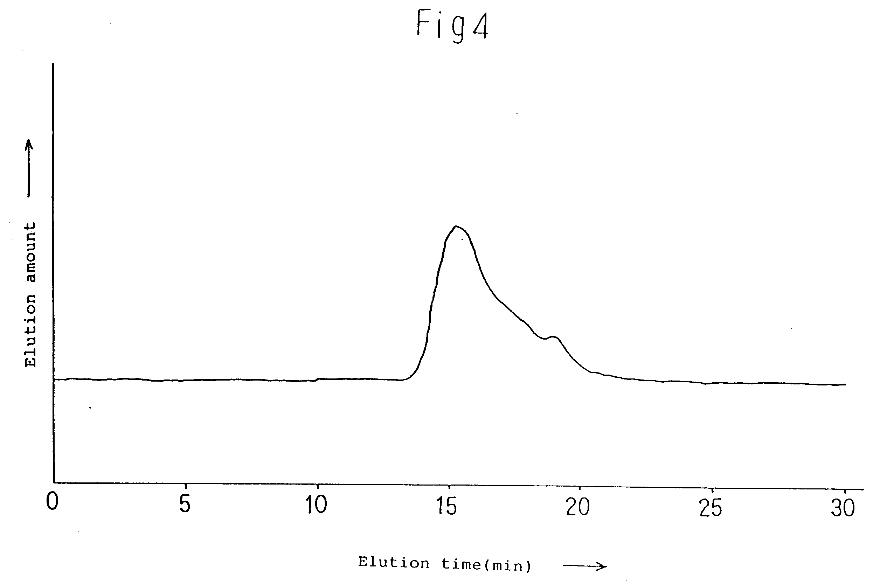

As a result, an elution curve as shown in Fig. 4 was obtained.

As explained above, according to the method of the present invention, for measuring a molecular weight distribution, and a spectrophotodetector of the present invention, a substance which is not melted until the temperature is raised to as high as, for example, 150°C, can be easily and accurately analyzed, and environmental pollution does not occur.

Further, according to an apparatus of the present invention, for measuring a distribution of a molecular weight of polymers, polymers dissolved in an elute having a high temperature can be accurately measured, using the above-mentioned spectrophotodetector, while maintained at a constant temperature.

## Claims

1. A method for measuring a molecular weight distribution by high performance liquid chromatography, wherein light permeated through a liquid sample in a cell is received at a light detecting member and wherein the sample is analyzed by outputting a chromatogramm on the basis of a signal corresponding to the received light,
said method being characterized in that the sample passing through the cell is heated to an extent such that the sample is not sedimented, and that the light which has been passed through the sample is received at a distance apart from the cell sufficient to avoid an influence of the temperature of the cell and/or the sample on the measurement.

2. A spectrophotodetector, comprising a cell (1) into which a liquid sample is introduced, a spectrophotodetecting member (11) arranged apart from the cell (1), a light transmitting means (14) which optically connects the spectrophotodetecting member (11) and the cell (1), and a means (8,9) for adjusting the temperature of the sample in the cell,
said spectrophotodetector being characterized in that said light transmitting means comprises an optical fiber (14) of a sufficient length such that the spectrophotodetecting member (11) is not affected by heat generated from the sample in the cell (1).

3. An apparatus for measuring a distribution of a molecular weight of polymers, comprising a means for supplying an elute, a means for introducing polymers as a sample into the supplied elute, a separating column which contains a packing material therein, and which separates and delivers the elute containing the polymers, and a spectrophotodetector including a cell into which the elute containing the polymers is introduced from the column,
said apparatus being characterized by a means (8, 9) for adjusting the temperature of the sample in said cell (1) and by a spectrophotodetecting member (11) which is arranged apart from the cell (1) to an extent such that said member is not affected by heat generated from at least a polymer-containing elute introduced into said cell (1), by means of a light transmitting means comprising an optical fiber (14) which optically connects the spectrophotodetecting member (11) and said cell (1).

## Patentansprüche

1. Verfahren zum Messen der Verteilung der Molekulargewichte durch Hochleistungs-Flüssigkeitschromatographie, bei dem Licht, welches durch eine flüssige Probe in einer Zelle hindurchgegangen ist, an einem Licht-Detektorelement empfangen wird und bei dem die Probe durch Ausgabe eines Chromatogramms auf der Basis eines Signals analysiert wird, welches dem empfangenen Licht entspricht, wobei das Verfahren dadurch gekennzeichnet ist, daß die die Zelle passierende Probe in einem solchen Ausmaß erwärmt wird, daß keine Sedimentation der Probe eintritt, und daß das Licht, welches durch die Probe hindurchgeleitet wurde, in einem solchen Abstand von der Zelle empfangen wird, daß ein Einfluß der Temperatur der Zelle und/oder der Probe bei der Messung vermieden wird.

2. Spektrumsfotodetektor mit einer Zelle (1), in die eine flüssige Probe eingeführt wird, mit einem Spektrum-Fotodetektorelement (11), welches im Abstand von der Zelle (1) angeordnet ist, mit Lichtübertragungseinrichtungen (14), die das Spektrums-Fotodetektorelement (11) optisch mit der Zelle (1) verbinden, und mit Einrichtungen (8, 9) zum Einstellen der Temperatur der Probe in der Zelle, wobei der Spektrums-Fotodetektor dadurch gekennzeichnet ist, daß die Lichtübertragungseinrichtungen eine optische Faser (14) mit einer ausreichenden Länge umfassen, derart, daß das Spektrums-Fotodetektorelement (11) nicht durch Wärme von der Probe in der Zelle (1) beeinflupt wird.

3. Vorrichtung zum Messen der Verteilung der Molekulargewichte von Polymeren, welche umfapt: Einrichtungen zum Liefern eines Eluenten, Einrichtungen zum Einleiten von eine Probe bildenden Polymeren in den zugeführten Eluenten, eine Trennkolonne, welche darin ein Packungsmaterial enthält und welche das Eluat, welches die Polymere enthält, abscheidet und liefert, und einen Spektrums-Fotodetektor, der eine Zelle enthält, in die das die Polymere enthaltende Eluat aus der Kolonne eingeleitet wird, wobei die Vorrichtung gekennzeichnet ist: durch Einrichtungen (8, 9) zum Einstellen der Temperatur der Probe in der Zelle (1) und durch ein Spektrums-Fotodetektorelement (11), welches in einem solchen Ausmaß im Abstand von der Zelle (1) angeordnet ist, daß es nicht von der Wärme beeinflußt wird, die von mindestens einem polymerhaltigen Eluat erzeugt wird, welches in die Zelle (1) eingeleitet wird, und zwar mit Hilfe von Lichtübertragungseinrichtungen, die eine optische Faser (14) umfassen, die das Spektrums-Fotodetektorelement (11) und die Zelle (1) optisch miteinander verbindet.

## Revendications

1. Procédé pour mesurer une distribution de masse moléculaire par chromatographie liquide à haute performance, dans lequel la lumière qui a traversé un échantillon liquide dans une cuve est reçue au niveau d'un élément détecteur de lumière et dans lequel l'échantillon est analysé par production d'un chromatogramme sur la base d'un signal correspondant à la lumière reçue, ledit procédé étant caractérisé en ce que l'échantillon traversant la cuve est chauffé dans une mesure telle que l'échantillon ne sédimente pas, et en ce que la lumière qui a traversé l'échantillon est reçue à une distance de la cuve suffisante pour éviter une influence de la température de la cuve et/ou de l'échantillon sur la mesure.

2. Spectrophotodétecteur comprenant une cuve (1) dans laquelle est introduit un échantillon liquide, un élément de spectrophotodétection (11) agencé à distance de la cuve (1), un moyen transmettant la lumière (14) qui relie optiquement l'élément de spectrophotodétection (11) et la cuve (1), et un moyen (8, 9) pour ajuster la température de l'échantillon dans la cuve, ledit spectrophotodétecteur étant caractérisé en ce que ledit moyen transmettant la lumière comprend une fibre optique (14) d'une longueur suffisante pour que l'élément de spectrophotodétection (11) ne soit pas affecté par la chaleur dégagée par l'échantillon dans la cuve (1).

3. Appareil pour mesurer une distribution de masse moléculaire de polymères, comprenant un moyen pour fournir un éluant, un moyen pour introduire des polymères en tant qu'échantillon dans l'éluant fourni, une colonne de séparation qui contient à l'intérieur un matériau de garnissage et qui sépare et fournit l'éluant contenant les polymères, et un spectrophotodétecteur comprenant une cuve dans laquelle l'éluant contenant les polymères est introduit depuis la colonne, ledit appareil étant caractérisé par un moyen (8, 9) pour ajuster la température de l'échantillon dans ladite cuve (1) et par un élément de spectrophotodétection (11) qui est agencé à distance de la cuve (1) dans une mesure telle que ledit élément n'est pas affecté par la chaleur dégagée par au moins un éluant contenant des polymères introduit dans ladite cuve (1), au moyen d'un moyen transmettant la lumière comprenant une fibre optique (14) qui relie optiquement l'élément de spectrophotodétection (11) et ladite cuve (1).
